# EUROPEAN PATENT SPECIFICATION

(11) **EP 0 659 072 B1**
(45) Date of publication and mention of the grant of the patent: **07.11.2001**
(21) Application number: 94920633.8
(22) Date of filing: 01.07.1994
(51) Int. Cl.: A61K 9/107

(54) **PHARMACEUTICAL EMULSIONS CONTAINING BIOACTIVE STEROIDS**
PHARMAZEUTISCHE EMULSIONEN, DIE BIOAKTIVE STEROIDE ENTHALTEN
EMULSIONS PHARMACEUTIQUES CONTENANT DES STEROIDES BIOACTIFS

(30) Priority: 02.07.1993 SE 9302295
(43) Date of publication of application: 28.06.1995
(73) Proprietor: Pharmacia AB, 112 87 Stockholm (SE)
(72) Inventor: JONASSON, Hallgrimur, S-175 60 Järfälla (SE); LI, Puyong, S-163 72 Spanga (SE); STENSTRÖM, Thomas, S-126 49 Hägersten (SE); ROSENQVIST, Kenneth, S-184 61 Akersberga (SE); MALINIAK, Ann, S-171 42 Solna (SE)
(86) International application number: SE9400656
(87) International publication number: WO9501162

(56) References cited:
- EP-A- 0 233 849
- EP-A- 0 391 369
- EP-A- 0 429 248
- DE-A- 2 162 593
- FIEDLER H P, "Lexikon der Hilfsstoffe für Pharmazie, Kosmetik und angrenzende Gebiete", 3. ed., 1989, pages 1051-1052, Editio Canter, Aulendorf
- FIEDLER: "Lexikon der Hilfsstoffe für Pharmazie, Kosmetik und angrenzende Gebiete", 1989, EDITIO CANTER, AULENDORF
- KIRK-OTHMER: "Encyclopedia of chemical technology, 3. ed. vol. 5", 1979, JOHN WILEY & SONS, NEW YORK

## Description

The present invention relates to novel oil-in-water emulsions especially suitable for parenteral administration of bioactive steroids which otherwise are difficult to solubilize. The emulsions will comprise a lipid phase containing castor oil that can comprise complementary lipids such as at least one vegetable oil and/or medium chain triglycerides.

### Background of the invention

Many therapeutically useful steroids are noted to have poor solubility in conventional pharmaceutical vehicles or solvents and it is attempted to increase their solubility, for example by introducing solubility modifying groups or by improving the properties of the vehicles for administration.

The British Patent Specifications GB 1 317 184 and GB 1 379 730 disclose anaesthetic compositions for the delivery of 3a-hydroxy-5a-pregnane-11,20-dione. These compositions which were accomplished as parenteral preparations of steroids together with a non-ionic surface active polyoxyethylated castor oil (Cremophor-EL) had, however, serious side-effects.

The European patent EP 0 233 849 discloses parenterally administerable lipid emulsions of 3a-hydroxy-5β-pregnan-20-one (pregnanolone), wherein the active steroid is dissolved in a lipid phase selected from coconut oil, borage oil, safflower oil, cotton seed oil, soybean oil and synthetic glycerides, having a mixture of long and medium chain fatty adds in the molecule.

Castor oil has previously been suggested as a vehicle for parenteral administration of steroid hormones together with organic co-solvents, such as benzyl alcohol and benzyl benzoate, as disclosed by C Riffkin et al in J. of Pharm. Sci., Vol. 53(8), 1964, pag. 891-5, or according to the American patent specification US 4 048 310, as a vehicle in topical preparations. The suggested co-solvents according to Riffkin et al., would, however, hardly be accepted as ingredients in a pharmaceutical preparation for parenteral use today.

The European patent application EP 0 418 004 discloses fat emulsions as carriers for compounds having prostaglandin E1 activities, which have a lipid phase containing an oil chosen from soybean oil, sesame oil, castor oil, cotton seed oil or olive oil. These emulsions will, however, beside a phospholipid emulsifier also contain an emulsifying adjuvant, preferably in the form of free fatty adds and a stabilizer. The level of free fatty adds is generally attempted to be kept as low as possible in parenteral emulsions in order to avoid the adverse effects disclosed in e.g. The Lancet, April 18 1970, pages 813 to 815 (V.A. Kurien et al.) and in EP 0 241 553.

Despite the disclosures of its usefulness as a solvent for poorly soluble pharmaceuticals, castor oil has in practice not been used in parenteral emulsions, because its high viscosity often leads to practical problems and because of its connection with the side-effects attributed to the castor oil derivatives contained in Cremophor-EL and possibly also its connection with the extremely potent toxin, ricin, naturally present in the castor oil plant.

There is a demand for an emulsion vehicle adapted to bioactive steroids, wherein both the solubility of the steroid compounds, which is limited in conventional vegetable oils, and the stability during autoclavation and storage is improved. It would be especially desirable to provide such a pharmaceutical emulsion containing only emulsifiers, solubilisers and other additional agents that are well disclosed as dinically acceptable and in suitable amounts. It would therefore be advantageous to avoid commonly employed synthetic solubilizers to improve the emulsion properties, since they often have undesired side effects. Pluronic F-68 has been shown to possess significant cardiovascular effects in dogs, and to cause lung and kidney alterations in rats. Tween-80 produces a wide range of CNS effects in rats and mice, causes histamine release and hypotension in dogs, and depresses myocardial contractility in guinea-pig heart preparations. Furthermore, the above mentioned Cremophor E1 is a potent histamine releasing agent dogs and is suspected to induce anaphylactoid reactions in humans, when used as a solvent for anaesthetic drugs.

In accordance with the present invention it is provided an oil-in-water emulsion suitable for administration of bioactive steroids with limited solubility in conventional solvents with a lipid phase comprising castor oil and an egg yolk phospholipid emulsifier.

It is the object of the present invention to provide a composition in the form of an oil-in-water emulsion which can contain higher amounts of dissolved steroids in the lipid phase with an improved physical and chemical stability and thereby reduce the total lipid load and the amount of solubilizers or stabilizers administered to the patient.

It is also the object of the present invention to provide an emulsion containing castor oil, optionally with an additional lipid, that has an excellent stability and retain suitable characteristics for parenteral administration of steroids after long-term storage, while comprising only well-disclosed biologically acceptable conventional constituents.

The inventive emulsion intends to overcome the previous emulsifying and stability problems experienced with castor oil and to provide an improved lipid emulsion vehicle for bioactive steroids that comprise clinically well-disclosed and safe constituents.

### Description of the invention

The present invention relates to a novel oil-in-water emulsion suitable for administration of bioactive steroids with a limited solubility in conventional solvents, having a lipid phase comprising castor oil, and egg yolk phospholipids as an emulsifier. The lipid phase can also further comprise at least one additional lipid selected from vegetable oils and/or synthetic or purified medium chain triglycerides. The invention is also directed to a process for the preparation of the said emulsions and their use in the preparation of an agent with an anaesthetic effect.

The lipids of inventive emulsion will contain castor oil of a pharmaceutically acceptable purity that may be combined with at least one complementary lipid selected from vegetable oils such as soybean oil, safflor oil, sesame oil, cotton seed oil or olive oil, and synthetic or purified medium chain triglycerides having fatty acid residues with between 6 and 12 carbon atoms in the molecule, such as MCT-fractions derived from coconut oil. Mixtures of the mentioned lipids are also suitable as an additive to the castor oil. The emulsifier is selected from clinically acceptable egg yolk phospholipids according to well established methods.
The relative amount of castor oil to other lipids are preferably within range of 90 : 10 % (w/w) to 10: 90 % (w/w) of castor and complementary lipids, respectively.

Suitable lipid compositions will contain castor oil only, or be mixtures of 70 % (w/w) of castor oil and 30 % (w/w) of soybean oil. The complementary lipids will be added in such an amount that the solvent capacity of the castor oil essentially is maintained. The relative amount of lipids is variable due to the polarity (chain length) of the added lipid constituents. To find suitable relative amounts for each lipid mixture of castor oil and complementary lipids to obtain a stable emulsion with the desired solubilizing properties will be within the experimental skill for persons familiar with this type of technology.
The high solubility of the steroids in castor oil might be explained by the hydroxylated fatty acids present (ricinoleic acid) which contribute to a higher polarity and to an increased solvent capacity.

The bioactive steroids to be administered with emulsions or compositions according to the present invention have a limited solubility in conventional hydrophilic and hydrophobic solvents and will preferably have activity on the central nervous system (CNS). Especially preferred are those CNS-active steroids which have an anaesthetic activity. The steroids most preferred according to the invention are selected from a group containing 5β-pregnan-3α-ol-20-one (eltanolone or pregnanolone), 5β-pregnan-3β-ol-20-one (epipregnanolone), 5α-pregnan-3α-ol-20-one (allopregnan-3α-ol-20-one), 5α-pregnan-3β-ol-20-one (allopregnanolone), 5α-pregnan-3α-ol-11,20-dione (alfaxalone), 5β-pregnan-3α,21-diol-20-one (tetrahydrodeoxycorticosterone or THDOC), 5α-pregnan-3α,21-diol-20-one (allotetrahydrodeoxycorticosterone or alloTHDOC), 5α-androstan-3α-ol-17-one (androsterone; cis-androsterone), 5-pregnen-3β-ol-20-one-sulphate (pregnenolone sulphate), 11β-11, 17, 21-trihydroxypregna-1,4-diene-3,20-dione (prednisolone) and 11β,17α,21-trihydroxypregna-4-ene-3,20-dione (dihydrocortisone). The steroids will be contained in an amount of 0.1 to 15 /ml in the emulsions provided by the present invention.

Lipids will be contained in the emulsions in an amount of 50-200 mg/ml and purified egg yolk phospholipids in an amount of 5 - 50 mg/ml.

A suitable emulsion invention will comprise:
a) a bioactive steroid in an amount of 5 mg/ml,
b) lipids containing castor oil and additional lipids in an amount of 10 % (w/w) of the total lipid emulsion,
c) an emulsifying agent in an amount of 1 to 5 g phospholipids from egg yolk per 100 ml of the final composition
d) water for injection,
e) isotonicity adjuster,
f) sodium hydroxide for pH adjustment.
The lipid phase of the emulsion will preferably contain castor oil in an amount of 10-90 % (w/w).
A suitable isotonicity adjuster is glycerol. If desired the emulsion can also contain conventional, but clinically acceptable, antioxidants and preservatives.

For preparing the novel oil-in-water emulsions, it is suitable to dissolve, in a first step, the bioactive steroid substance in the lipids. The resulting solution is then emulsified by means of conventionally used high pressure homogenizers, in an aqueous medium comprising water for injection, emulsifying agent and isotonicity adjuster as described above and, if desired, any of the specified additional components. In the resulting emulsion, the particle size of the oil droplets will be less than 5 µ, with a large part less than 1 µ, preferably from 0.2 to 0.3 µ.

The novel oil-in-water emulsions will essentially comprise a solution of a bioactive steroid in a lipid phase, where the lipid phase is emulsified in a hydrophilic phase. These emulsions will be especially useful for parenterally administering an anaesthetically active steroid to a patient.

The emulsions according to the present invention have an excellent chemical and physical stability with a minimized oxidation, due to the inherent characteristics of the castor oil itself, and a reduced tendency of droplet coalescence with following creaming effects, as well as a low level of free fatty acid formation. The emulsions have also the capability of withstanding autoclaving procedures. Because of the higher level of solubility of the steroids, the problem with precipitations on various surfaces will be reduced, which allows manufacturers more freedom in the choice of suitable packages. The inventive emulsions will also be more compatible with conventional plastic and rubber articles used in medical applications.
The high solvent capacity and the improved stability of the emulsion will also enable an exclusion of several traditionally used, but in certain aspects controversial additives, such as Cremophor-EL, polyethylene glycol, propylene glycol, benzyl alcohol, benzyl benzoate and ethanol.
Another advantage is the reduction of lipids in the novel emulsions, which will lead to a low lipid load for the patient during the administration.
Further advantages are apparent from the following examples and will include such important properties for an anaesthetically active steroid (eltanonolone) administered with the inventive emulsion, as less cardiovascular effects.

Example 1 shows the preparation of a number of emulsions according to the present invention.
Example 2 shows the compostion of emulsions according to the present invention that are biologically tested in Examples 3 to 5.
Example 3 shows the anaesthetic effect of eltanolone administered in the inventive emulsions compared to a state of the art emulsion.
Example 4 shows comparisons of cardiovascular effects.
Example 5 shows an investigation of the acute toxicity.

### Example 1

In order to determinate the quality of the castor oil containing emulsions with different steroids, the following compositions were prepared:

| | |
|---|---|
| Steroid | 0.10 g |
| Oil phase | 20.0 g |
| Phospholipids | 2.40 g |
| Glycerol | 4.50 g |
| NaOH (1M) | 0.70 g |
| Water for injection | 172.3 g |

The steroids were weighed in a 50 ml glass beaker. The castor oil (CO) or a mixture of castor oil and soybeans oil (CO+SBO) in a proportion of 70 : 30 was added to the beaker which was heated and the steroids were dissolved. The phospholipids were added to the oil phase and the agitation continued for another five minutes. This mixture was added to a heated aqueous phase containing water and glycerol and agitated for five minutes. The mixture was thereafter homogenized to an emulsion in a high pressure lab scale homogeniser at the technically highest possible pressure. The resultant emulsions were dispersed and sealed 20 ml glass vials which were heat sterilized. The emulsions were analysed with respect to the appearance, the shaking stability, pH and volume distribution. The span was analysed with a Malvern Mastersizer. The appearance of the droplets larger than 1µm and the average area on a relevant picture and the presence of precipitate were investigated with a microscope. The storage stability at 5 and 40°C was also studied.

| Substance | Vehicle | pH | D(4:3) µm | Span | Storage 5 and 40°C |
|---|---|---|---|---|---|
| 1. Alfaxalone | CO | 8.9 | 0,83 | 1.85 | >4 months |
| | CO+SBO | 8.9 | 0.58 | 1.61 | >4 months |
| 2.Epipreganolone | CO | 8.8 | 0.75 | 1.81 | >4 months |
| | CO+SBO | 9.0 | 0.75 | 1.71 | >4 months |
| 3. Allo | CO | 8.8 | 0.69 | 1.70 | >3 months |
| pregnanolone | CO+SBO | 9.0 | 0.53 | 1.58 | >3 months |
| 4.Tetrahydrode | CO | 9.0 | 0.86 | 1.80 | >1 month |
| oxycorticosterone | CO+SBO | 9.1 | 0.58 | 1.52 | >1 month |
| 5. Pregnenolone | CO | 8.8 | 0.97 | 1.76 | >1 month |
| sulphate | CO+SBO | 8.8 | 0.50 | 1.56 | >1 month |
| 6. Androsterone | CO | 8.9 | 0.83 | 1.79 | >1 month |
| | CO+SBO | 9.2 | 0.51 | 1.50 | >1 month |
| 7. Prednisolone | CO | 8.9 | 0.81 | 1.28 | >2 months |
| | CO+SBO | 8.9 | 0.70 | 1.26 | >2 months |
| 8. Hydrocortisone | CO | 8.7 | 0.76 | 1.43 | - |
| | CO+SBO | 8.8 | 0.56 | 1.51 | - |

The results show that the emulsions with castor oil containing oil phase is a suitable vehicle for a wide range of poorly soluble steroids. The emulsions have generally a good stability during prolonged storage and suitable characteristics for parenteral administration.

### Example 2

For the biological experimentation oil-in-water emulsions were prepared from the following components:

| | Formulation 1 (mg) | Formulation 2 (mg) | Formulation 3 (mg) |
|---|---|---|---|
| Eltanolone (INN) | 4 | 4 | 4 |
| Castor oil | 70 | 70 | - |
| Soybean oil | 30 | - | 200 |
| MCT | | 30 | |
| Diacetyl. monoglyc. | - | - | 70 |
| Phospholipids | 12 | 12 | 18 |
| Glycerol | 22.5 | 22.5 | 17 |

pH was adjusted to 8.5 by NaOH and water for injection was added to 1 ml in all formulations.

Formulations 1 and 2 are emulsions according to the present invention, whereas Formulation 3 is a state of the art emulsion according to the European patent
EP 233 849. The medium chain triglycerides in Formulation 2 are derived from coconut oil and will contain a fraction of triglycerides with fatty add residues having 6 to 12 carbon atoms.
According to the present invention, the eltanolone was first mixed with castor oil or a mixture of castor oil and soybean oil and/or MCT. This leads to a substantial dissolution of the eltanolone. An emulsion was then prepared from the resulting oil-eltanolone mixture together with the additional indicated components. The resulting emulsions were stable and had average particle sizes of about 0.2 to 0.3 µ and could be sterilized by autoclaving without destabilisation.
It is notable that in Formulations 1 and 2 according to the present invention half the amount of lipids is sufficient for obtaining a substantial dissolution of eltanolone when compared to Formulation 3. The solubility of eltanolone is for example about 5 times higher in Formulation 1 than in Formulation 3.

The same emulsion as in Formulation I is thereafter prepared with 4 mg/ml of 5β-pregnan-3β-ol-20-one (epipregnanolone) instead of eltanolone with similar results in solubility of the steroid and emulsion stability.

### Example 3

### Determination of the anaesthetic effect

Formulations 1 and 3 according to Example 2 were compared with regard to anaesthetic effect. For this experiment 30 Sprague-Dawley male rats with body weights ranging between 343 and 391 grams were used. The rats were prepared with EEG electrodes consisting of fourfold twined stainless steel threads (0.2 mm) which were fastened subcutaneously in front of each ear.
The emulsions were administered as an intravenous infusion via a tail vein, at a dose infusion rate of 2 mg/kg/min. The EEG was monitored continuously, and as soon as an EEG burst suppression of at least one second (a "silent second") was observed, the infusion was terminated. The animal was then laid on its side in its cage and observed until it regained its righting reflex.
The measured parameters were:
- The dose of eltanolone needed to induce the silent second.
- The sleeping time i.e. the time from the appearance of the silent second until the return of the righting reflex

Results (group mean values±SE):

| Emulsion | Dose needed to induce the silent second (mg/kg) | Sleeping time (min) |
|---|---|---|
| Formulation 1 | 8.75±0.38 | 43±2 |
| Formulation 3 | 8.73±0.55 | 28±1 |

The results show that, at the dose infusion rate employed, there was no difference between the emulsions as regards induction of cortical EEG silence (a silent second), while the sleeping time was somewhat longer after administration of Formulation 1.

### Example 4

### Cardiovascular investigation

The purpose of this investigation was to compare Formulations 1 and 3 according to Example 2 regarding cardiovascular effects. Five chronically instrumented dogs were used in this investigation. This model allows registration of cardiovascular parameters in the absence of surgical stress and additional drugs, such as premedication and basal anaesthetics. The transition from the awake state to anaesthesia and to awakening could thereby be followed without interference. Both emulsions were tested in the same animal on different occasions. The dose of eltanolone was 5 mg/kg given as a bolus intravenous injection. This dose is approximately twice the amount normally required for inducing anaesthesia in a dog.

The most significant findings were:

| Parameter | Approximate maximum change (%) from the awake state | |
|---|---|---|
| | Formulation 1 | Formulation 3 |
| Heart rate | +20 | +100 |
| Mean arterial blood pressure | ±0 | -20 |
| Stroke volume | -20 | -65 |
| Left ventricular dP/dt max | -10 | -50 |
| (force of contraction) | | |

Anaesthesia induced with Formulation 1 was associated with substantially less cardiovascular effects than that induced with Formulation 3.

### Example 5

### Investigation of acute toxicity

Acute toxicity after an intravenous high dose (10 or 16 times the effective anaesthetic dose for rats) single administration of Formulation 1 was tested in rats. 40 male Sprague-Dawley rats with body weights ranging between 198 and 261 g were used. The sleeping time, i.e. the time between the loss and return of the righting reflex, was measured. During anaesthesia and after awakening, the animals were observed for clinical symptoms such as breathing pattern, heart rhythm and skin colour.
During a follow-up period of two weeks the animals' general condition was observed. The rats were weighed immediately before the administration, then on days seven and 14.

All animals survived the experiment. The doses, the infusion rates and results are shown in the table below.
During anaesthesia a somewhat lowered, but steady, respiration rate was observed. However, no signs of cyanosis were noted. Also the heart rate was somewhat reduced, but it was regular with a strong beat. Respiratory gurgling sounds were noted in some animals at the end of the sleeping period, but only one animal needed manual breathing assistance. A transient peripheral vasoconstriction was observed in all animals. Directly after awakening the animals seemed rather sluggish, but appeared otherwise to be in good condition. The behaviour of the rats and their general health was normal during the follow-up period, as well as their increase in weight.

| Group | Dose mg/kg | Dose ml/kg | Infus. rate. ml/min | Numb. animals | Sleeping Time min | Weight, g day 0 | Weight increase, g day 0-7 | Weight increase, g day 8-14 |
|---|---|---|---|---|---|---|---|---|
| A | 40 | 10 | 0,5 | 10 | 98±7 | 222±3 | 43±3 | 40±3 |
| B | 64 | 16 | 0,1 | 10 | 133±5 | 229±5 | 40±2 | 42±2 |
| C | 64 | 16 | 0,5 | 10 | 145±5 | 224±4 | 39±2 | 38±2 |
| D | 64 | 16 | 2,5 | 10 | 151±6 | 229±5 | 37±1 | 39±2 |
| Sleeping time and weights are registered as mean values ±SE | | | | | | | | |

The doses, infusion rates and the sizes of the rats were in accordance with previously performed acute toxicity tests for intravenous anaesthetics.
The results are satisfying and demonstrate that an emulsion according to the present invention shows no toxic effects.

## Claims

1. An oil-in-water emulsion suitable for parenteral administration of bioactive steroids with limited solubility in conventional solvents **characterized in that** it comprises per ml of the final composition:
a) steroid in an amount of 0.1 to 15 mg;
b) lipids in an amount of 50 to 200 mg comprising castor oil and optionally at least one additional lipid selected from vegetable oils and/or synthetic or purified medium chain triglyceride;
c) an emulsifier in an amount of 5 to 50 mg selected from purified egg yolk phospholipids;
d) water for injection;
e) an isotonicity adjuster added to isotonicity of the final composition; and
f) sodium hydroxide solution added to a suitable pH, while excluding additives selected among polyethylene glycol, Pluronic® F-68, benzyl alcohol and benzyl benzoate.

2. An emulsion according to claim 1 **characterized in that** the steroids are selected from a group consisting of 5β-pregnan-3α-ol-20-one (eltanolone or pregnanolone), 5β-pregnan-3β-ol-20-one (epipregnanolone), 5α-pregnan-3α-ol-20-one (allopregnan-3α-ol-20-one), 5α-pregnan-3β-ol-20-one (allopregnanolone), 5α-pregnan-3α-ol-11,20-dione (alfaxalone), 5β-pregnan-3α,21-diol-20-one (tetrahydrodeoxycorticosterone or THDOC), 5α-pregnan-3α,21-diol-20-one (allotetrahydrodeoxycorticosterone or AlloTHDOC), 5α-androstan-3α-ol-17-one (androsterone or cis-androsterone), 5-pregnen-3β-ol-20-one-sulphate (pregnenolone sulphate), 11β-11,17,21-trihydroxypregna-1,4-diene-3,20-dione (prednisolone) and 11β,17α,21-trihydroxypregna-4-ene-3,20-dione (dihydrocortisone)

3. An emulsion according to claim 1 **characterized in that** the lipids contain 10 to 90 % (w/w) castor oil.

4. An emulsion according to claim 3 **characterized in that** the lipids contain 70 % (w/w) castor oil and 30 % (w/w) soybean oil.

## Patentansprüche

1. Öl-in-Wasser-Emulsion, die für eine parenterale Verabreichung von bioaktiven Steroiden mit begrenzter Löslichkeit in herkömmlichen Lösemitteln geeignet ist, **dadurch gekennzeichnet, dass** sie pro ml der endgültigen Zusammensetzung enthält:
a) Steroid in einer Menge von 0,1 bis 15 mg;
b) Lipide in einer Menge von 50 bis 200 mg, umfassend Rizinusöl und gegebenenfalls mindestens ein zusätzliches Lipid, ausgewählt aus pflanzlichen Ölen und/oder synthetischem oder gereinigtem Triglycerid mit mittlerer Kettenlänge;
c) ein Emulgiermittel in einer Menge von 5 bis 50 mg, ausgewählt aus gereinigten Eigelb-Phospholipiden;
d) Wasser für die Injektion;
e) ein Mittel zum Anpassen der Isotonie, das bis zur Isotonie der endgültigen Zusammensetzung zugesetzt wird; und
f) Natriumhydroxidlösung, die bis zu einem geeigneten pH zugesetzt wird, wobei Zusatzstoffe, ausgewählt unter Polyethylenglycol, Pluronic® F-68, Benzylalkohol und Benzylbenzoat, ausgeschlossen sind.

2. Emulsion nach Anspruch 1, **dadurch gekennzeichnet, dass** die Steroide ausgewählt sind aus einer Gruppe, bestehend aus 5β-Pregnan-3α-ol-20-on (Eltanolon oder Pregnanolon), 5β-Pregnan-3β-ol-20-on (Epipregnanolon), 5α-Pregnan-3α-ol-20-on (Allopregnan-3α-ol-20-on), 5α-Pregnan-3β-ol-20-on (Allopregnanolon), 5α-Pregnan-3α-ol-11,20-dion (Alfaxalon), 5β-Pregnan-3α,21-diol-20-on (Tetrahydrodeoxycorticosteron oder THDOC), 5α-Pregnan-3α,21-diol-20-on (Allotetrahydrodeoxycorticosteron oder AlloTHDOC), 5α-Androstan-3α-ol-17-on (Androsteron oder cis-Androsteron), 5-Pregnen-3β-ol-20-on-sulfat (Pregnenolonsulfat), 11β-11,17,21-Trihydroxypregna-1,4-dien-3,20-dion (Prednisolon) und 11β,17α,21-Trihydroxypregna-4-en-3,20-dion (Dihydrocortison).

3. Emulsion nach Anspruch 1, **dadurch gekennzeichnet, dass** die Lipide 10 bis 90% (Gew./Gew.) Rizinusöl enthalten.

4. Emulsion nach Anspruch 3, **dadurch gekennzeichnet, dass** die Lipide 70% (Gew./Gew.) Rizinusöl und 30% (Gew./Gew.) Sojabohnenöl enthalten.

## Revendications

1. Emulsion huile dans l'eau convenant pour l'administration parentérale de stéroïdes bioactifs à solubilité limitée dans les solvants conventionnels **caractérisée en ce qu'**elle comprend par ml de la composition finale :
a) un stéroïde en une quantité de 0,1 à 15 mg ;
b) des lipides en une quantité de 50 à 200 mg comprenant de l'huile de ricin et éventuellement au moins un lipide supplémentaire choisi parmi les huiles végétales et/ou un triglycéride à chaîne moyenne synthétique ou purifié ;
c) un émulsifiant en une quantité de 5 à 50 mg choisi parmi les phospholipides du jaune d'oeuf purifiés ;
d) de l'eau pour injection ;
e) un agent d'ajustement de l'isotonie ajouté jusqu'à l'isotonie de la composition finale ; et
f) une solution d'hydroxyde de sodium ajoutée jusqu'à un pH approprié, à l'exclusion des additifs choisis parmi le polyéthylèneglycol, le Pluronic® F-68, l'alcool benzylique et le benzoate de benzyle.

2. Emulsion selon la revendication 1
**caractérisée en ce que** les stéroïdes sont choisis dans un groupe consistant en la 5β-prégnan-3α-ol-20-one (eltanolone ou prégnanolone), la 5β-prégnan-3β-ol-20-one (épiprégnanolone), la 5α-prégnan-3α-ol-20-one (alloprégnan-3α-ol-20-one), la 5α-prégnan-3β-ol-20-one (alloprégnanolone), la 5α-prégnan-3α-ol-11,20-dione (alfaxalone), la 5β-prégnane-3α,21-diol-20-one (tétrahydrodésoxycorticostérone ou THDOC), la 5α-prégnane-3α,21-diol-20-one (allotétrahydrodésoxycorticostérone ou AlloTHDOC), la 5α-androstan-3α-ol-17-one (androstérone ou cis-androstérone), le sulfate de 5-prégnén-3β-ol-20-one (sulfate de prégnénolone), la 11β-11,17,21-trihydroxyprégna-1,4-diène-3,20-dione (prednisolone) et la 11β,17α,21-trihydroxyprégna-4-ène-3,20-dione (dihydrocortisone).

3. Emulsion selon la revendication 1
**caractérisée en ce que** les lipides contiennent 10 à 90 % (m/m) d'huile de ricin.

4. Emulsion selon la revendication 3
**caractérisée en ce que** les lipides contiennent 70 % (m/m) d'huile de ricin et 30 % (m/m) d'huile de soja.
